# EUROPÄISCHE PATENTSCHRIFT

(11) **EP 0 462 422 B1**
(45) Veröffentlichungstag und Bekanntmachung des Hinweises auf die Patenterteilung: **05.10.1994**
(21) Anmeldenummer: 91108529.8
(22) Anmeldetag: 25.05.1991
(51) Int. Cl.: A61M 1/30

(54) **Verfahren und Vorrichtung zum Reinigen des Blutes von Stoffwechselprodukten in einer Singel-Needle-Anordnung**
Method and device for purification of blood from metabolic products in a single needle system
Procédé et dispositif de purification du sang des produits de métabolisme dans un système à aiguille unique

(30) Priorität: 12.06.1990 DE 4018723
(43) Veröffentlichungstag der Anmeldung: 27.12.1991
(73) Patentinhaber: Fresenius AG, 61350 Bad Homburg (DE)
(72) Erfinder: Polaschegg, Hans-Dietrich, Dr., W-6370 Oberursel 4 (DE)
(74) Vertreter: Fuchs Mehler Weiss

(56) Entgegenhaltungen:
- EP-A- 0 088 900
- DE-A- 3 131 075
- US-A- 3 756 234

## Beschreibung

Die Erfindung betrifft ein Verfahren und eine Vorrichtung zum Reinigen des Blutes von Stoffwechselprodukten in einem extracorporalen Kreislauf in einer Single-Needle-Anordnung.

Die Verwendung einer Single-Needle-Anordnung oder einer Anordnung mit einer Einzelnadel bei der Dialyse ist seit langem bekannt.

Im Gegensatz zu der üblichen Hämodialyse, bei der ein Patient jeweils mit einer Nadel für den Hin- und Rücklauf des Blutes punktiert wird, kommt man bei der Single-Needle-Dialyse mit nur einer Nadel aus. Diese Methode hat den Vorteil, daß sich die Zahl der Punktionen auf die Hälfte reduziert, sodaß einerseits die Gefäße geschont und andererseits die bei der Punktion auftretenden Schmerzen vermindert werden.

Dieser Vorteil ist beispielsweise bereits in der US-PS 3756234 hervorgehoben. Darin ist ein extracorporaler Dialysekreislauf beschrieben, welcher in der Single-Needle-Anordnung betrieben wird, sowie ein entsprechendes Verfahren.

Das bekannte Verfahren hat den Vorteil, daß eine Single-Needle-Behandlung mit den gleichen Komponenten des extracorporalen Kreislaufs durchgeführt werden kann wie eine Zwei-Nadel-Behandlung, da zu dessen Durchführung lediglich eine Blutpumpe und eine Klemme erforderlich sind, die üblicherweise vorhanden sind.

Bei dem Verfahren wird Blut durch die arterielle Blutpumpe durch den Dialysator in eine teilweise mit Luft gefüllte Tropfkammer, die stromab durch eine Klemme geschlossen ist, gefüllt. Dabei wird die Luft in dieser Tropfkammer komprimiert, wodurch der Druck in der Kammer steigt. Bei Überschreiten eines oberen Schaltpunktes wird die Pumpe durch eine sie ansteuernde Steuereinheit gestoppt und die Klemme geöffnet. Daraufhin entlädt sich der Druckspeicher, sodaß gereinigtes Blut zu dem Patienten zurückgefördert wird. Da dabei der Druck exponentiell abfällt, fällt auch der Fluß exponentiell ab, sodaß die Blutrückführung relativ lange dauert und der mittlere Blutfluß geringer als optimal erreichbar ist.

Um diesen Nachteil zu beheben, ist beispielsweise in der DE 3131075 A1 ein Speicher beschrieben worden, der in dem Dialysesystem arteriell angeordnet ist. Dessen Wände sind nach außen hin elastisch erweiterbar ausgebildet, sodaß sie im gefüllten Zustand unter Zugspannung stehen. Wird nun der Betrieb der Blutzuführpumpe gestoppt und die venöse Klemme geöffnet, fließt das Blut gesteuert aus dem Speicher durch die Dialysemembran zum Patienten.

Eine weitere Ausbildung eines derartigen Speichers ist im übrigen aus der DE 3205449 A1 bekannt.

Die erwähnten Speicher sollen dazu dienen, den Druck unabhängig vom Füll- bzw. Entleerungsgrad konstant zu halten, damit auch der Blutfluß zum Patienten hin konstant ist.

Wenngleich die bekannten Speicher diese Forderung im wesentlichen erfüllen, haftet ihnen als Einmalartikel der Nachteil an, daß der sich einstellende Druck durch die Auslegung des Speichers bestimmt und daher nicht veränderbar ist. Darüberhinaus ist die Konstruktion der Speicher relativ aufwendig. Dennoch haben die Anforderungen an den Speicher, der bei einer normalen Dialysesitzung viele tausend Male aufgeladen wird, wegen der zu fordernden Zuverlässigkeit dessen praktische Verwirklichung weitgehend verhindert.

Vor dem aufgezeigten Hintergrund ist es nun die Aufgabe der vorliegenden Erfindung, die aufgeführten Nachteile zu vermeiden. Es soll demgemäß ein Blutreinigungsverfahren in einem extracorporalen Kreislauf in Single-Needle-Anordnung angegeben werden, bei dem der venöse Blutrücklauffluß im wesentlichen konstant ist und welches ohne Schwierigkeiten auch in Dialysesystemen, die für ein Zwei-Nadel-Verfahren ausgelegt sind, ausführbar ist. Desweiteren soll eine Vorrichtung für die Durchführung des Verfahrens geschaffen werden.

Gelöst wird die Aufgabe hinsichtlich der Vorrichtung durch den kennzeichnenden Teil des Anspruchs 1. Die Aufgabe hinsichtlich des Verfahrens wird ausgehend von dem beschriebenen Verfahren durch den kennzeichnenden Teil des Anspruchs 14 gelöst.

Weitere vorteilhafte Ausführungsformen ergeben sich aus den jeweiligen Unteransprüchen.

Erfindungsgemäß wird der Blutspeicher zumindest während der venösen Blutflußphase von einer externen Einrichtung so mit Kraft beaufschlagt, daß sich in seinem Inneren ein im wesentlichen von seinem Füllvolumen unabhängiger Druck einstellt.

Während der arteriellen Blutflußphase, also wenn dem extracorporalen Kreislauf Blut mittels der Blutpumpe zugeführt wird und ein Blutrückfluß zum Patienten in Übereinstimmung mit dem bekannten Verfahren durch Schließen eines stromab gelegenen Absperrorgans verhindert wird, wird der elastische Blutspeicher mit Blut gefüllt.

Zur Einleitung der venösen Blutphase wird in Übereinstimmung mit den bekannten Verfahren der Betrieb der Blutpumpe gestoppt und das stromab gelegene Absperrorgan geöffnet. Der konstante Blutfluß aus dem Blutspeicher wird sodann dadurch erreicht, daß eine externe Einrichtung den Blutspeicher im dargelegten Sinne mit einer Kraft beaufschlagt. Die Kraft muß mit anderen Worten im wesentlichen konstant sein.

Gegenständlich gesprochen wird also der Blutspeicher während der arteriellen Blutflußphase nach Art eines Ballons aufgeblasen und während der venösen Blutflußphase durch die externe Einrichtung mit konstanter Kraft ausgedrückt.

Der aus dem Blutspeicher fließende Blutfluß ist aufgrund der beschriebenen Maßnahme im wesentlichen konstant. Dadurch, daß eine externe Einrichtung Verwendung findet, läßt sich das Verfahren leicht mit jeder üblichen Zwei-Nadel-Anordnung durchführen, da diese Einrichtung einfach zu den bestehenden Komponenten hinzugefügt werden kann.

Das Verfahren kann vorteilhaft dadurch ausgeführt werden, daß der Blutspeicher während der arteriellen Blutflußphase entgegen der von der externen Einrichtung erzeugten Kraft mit Blut gefüllt wird, wodurch die Einrichtung vorgespannt wird, und daß der Blutspeicher während der venösen Blutflußphase unter Abbau der Vorspannung entleert wird. Hierdurch wird in vorteilhafter Weise während des quasi Aufpumpens des Blutspeichers diejenige Kraft erzeugt, die zu seinem anschließenden Entleeren mit konstantem Fluß gefordert wird. Dies erleichtert die Konstruktion und Anwendung der externen Einrichtung erheblich, da kein kompliziertes Steuerungssystem vorgesehen werden muß.

Die erfindungsgemäße Vorrichtung weist neben den Komponenten der bekannten Vorrichtungen eine auf den Blutspeicher eine im wesentlichen konstante Kraft ausübende Einrichtung aus.

Im Unterschied zu den bekannten Vorrichtungen ist also eine aktive Komponente vorgesehen, die auf den Blutspeicher im beschriebenen Sinne einwirkt, während es den bekannten Speichern in einem Dialysekreislauf selbst überlassen blieb, sich aufgrund der im gefüllten Zustand herrschenden Zugkräfte selbst zu entleeren.

In einfacher Ausführung besteht die externe Einrichtung aus einer feststehenden Platte und aus einer gegenüber dieser beweglichen Platte, wobei die bewegliche Platte aufgrund einer auf sie einwirkenden Feder mit einer voreinstellbaren Kraft gegen die feststehende Platte gedrückt wird und der Blutspeicher zwischen den beiden Platten angeordnet ist. Die Feder sorgt dabei während der venösen Blutflußphase für die auf den Speicher einwirkende, im wesentlichen konstante wegunabhängige Kraft. Sie wird während der arteriellen Phase, also wenn sich der zunächst weitgehend entleerte Speicher zwischen den dann eng gegenüberstehenden Platten befindet, mit zunehmender Füllung des Speichers unter Vorspannung gesetzt, die während der venösen Phase abgebaut und zur Entleerung genutzt wird.

Die Verwendung dieser Einrichtung an einem Zwei-Nadel-System ist denkbar einfach. So wird sie einfach an das bestehende System angeordnet, sodaß sie auf den Blutspeicher einwirken kann. Vorzugsweise wird dies an einer Stelle stromab der Blutpumpe und stromauf des Absperrorgans der Fall sein.

Dadurch, daß die Kraft, mit der die bewegliche Platte gegen die feststehende Platte gedrückt wird, voreinstellbar ist, kann der Blutrückfluß individuellen Patientenerfordernissen angepaßt werden. Dies ist bei bekannten Speichern nicht möglich.

Der Blutspeicher selbst kann als elastische Kammer ausgebildet sein. Beispielsweise kann ein einfacher Beutel aus geeigneten Kunststoff oder ein Faltenbalg als Blutspeicher dienen. Der Speicher hat typischerweise ein Füllvolumen von 10 - 100 ml.

Eine alternative Ausführungsform der erfindungsgemäßen Vorrichtung ist so ausgestaltet, daß die auf den Blutspeicher Kraft ausübende Einrichtung aus zwei in einem Rahmen schwimmend gelagerten Platten besteht, von denen jede mittels jeweils einer auf sie einwirkenden Feder mit einer einstellbaren Kraft gegen die jeweils andere Platte gedrückt wird, wobei der Blutspeicher zwischen beiden Platten angeordnet ist. Auch hierbei kann der Blutspeicher vorteilhaft in Form einer elastischen Kammer ausgebildet sein.

Vorteilhaft, da kostensparend, ist es, wenn der Blutspeicher gleichzeitig die Funktion eines in jedem Dialysesystem vorhandenen Luftabscheiders wahrnimmt. Hierzu ist der Blutspeicher beispielsweise in Form eines Beutels mit zwei Anschlüssen für die Zu- und Abfuhr von Blut versehen. Eine weitere Öffnung im Beutel ist mit einer Hydrophobmembran verschlossen, die ein Entweichen von Luft erlaubt.

Vorteilhaft ist die externe Einrichtung mit einem Absolutweggeber versehen, der die Position der beweglichen Platte gegenüber der feststehenden Platte erfaßt. Das von dem Positionsmelder oder Absolutweggeber erzeugte Signal läßt sich dahingehend weiter verarbeiten, daß aus ihm das Füllvolumen des Blutspeichers bestimmt wird und ein entsprechendes Signal für die Steuerung des Single-Needle-Verfahrens herangezogen werden kann. Die Steuerung und Drucküberwachung in der erfindungsgemäßen Vorrichtung erfolgt im übrigen nach bekannten Gesichtspunkten, insbesondere hinsichtlich des Öffnens und Schließens des stromab des Blutspeichers gelegenen Absperrorgans sowie der Ansteuerung der Blutpumpe, auch wenn die moderne Elektronik eine gegenüber bekannten Vorrichtung ein wesentlich genaueres Schalten und Überwachen zuläßt.

Wenn die Vorrichtung ständig mit der externen Einrichtung versehen ist, also jeweils im konkreten Therapiefalle entschieden wird, ob das erfindungsgemäße Single-Needle-Verfahren oder aber das bekannte Zwei-Nadel-Verfahren angewandt werden soll, ist es zweckmäßig, die externe Einrichtung mit einer Arretierungsvorrichtung zu versehen, welche die bewegliche Platte gegenüber der feststehenden Platte festlegt. Hierdurch kann das Volumen des Blutspeichers begrenzt werden, um im Falle der Anwendung des Zwei-Nadel-Verfahrens das Füllvolumen des extracorporalen Kreislaufs nicht unnötig zu vergrößern.

Bei dem ersten Ausführungsbeispiel der externen Einrichtung, die aus einer feststehenden Platte und einer beweglichen Platte besteht, zwischen denen der Blutspeicher angeordnet ist, führen Überlegungen zur Erzielung eines konstanten Druckes des Blutes im Blutspeicher zu folgendem Ergebnis: Die Federkraft ist konstant, wohingegen die Druckfläche oder wirksame Fläche A der beweglichen Platte gegen den Blutspeicher mit einer maximalen Auflagefläche B bei minimalem Volumen nicht notwendigerweise konstant sein muß, infolge einer Ausbauchung. Als vorteilhaft hat es sich herausgestellt, die Größe der wirksamen Fläche der Platte so zu wählen, daß für das Verhältnis A:B gilt: 0,7 < a/b < 0,9, also die wirksame Fläche der Platte zwischen 70 und 90 % der Blutspeicherfläche bei minimalem Füllvolumen, die mit Kraft beaufschlagt wird, zu wählen.

Wenn die maximale Dicke des maximal befüllten Blutspeichers a beträgt und dieser in den anderen Dimensionen (Breite, Höhe) eine minimale Dimension b aufweist, dann ist es vorteilhaft, den Blutspeicher so auszubilden, daß a etwa 8 bis 12 % des Wertes der minimalen Dimension b beträgt.

Durch die angegebenen Größenverhältnisse ist ein nahezu konstanter Druck des Blutes im Blutspeicher erzielbar.

Bei der alternativen Ausführungsform der externen Einrichtung aus zwei schwimmend gelagerten Platten gilt hinsichtlich des Verhältnisses der wirksamen Fläche C der Platten zu der Auflagefläche D des Blutspeichers bei minimalem Füllvolumen Entsprechendes, also daß sich C:D verhalten wie 0,7 < c/d < 0,9.

Hinsichtlich der maximalen Dicke c des maximal befüllten Blutspeichers kann hier vorteilhaft die Verdoppelung des Verhältnisses gegenüber der ersten Ausführungsform treten, so daß also c:d sich verhalten wie 0,16 < c/d < 0,24, wobei d die minimale Dimension (Breite, Höhe) des Blutspeichers bedeutet.

In beiden Ausführungsformen der externen Einrichtung ist vorteilhaft ein einstellbarer Anschlag für die bewegliche Platte oder die beweglichen Platten vorgesehen, mittels dessen das maximale Füllvolumen des Blutspeichers festlegbar ist. Der Beutel kann ferner entlang seiner Schweißnaht durch eine Haltevorrichtung geklemmt werden. Hierdurch wird einer etwaigen Gefahr eines Platzens des Blutspeichers bei noch größeren Füllvolumina vorgebeugt.

Die Erfindung wird anhand von Ausführungsbeispielen gemäß der Zeichnungen näher erläutert. Hierbei zeigt:
- Figur 1: Schematisch eine Single-Needle-Dialyseanordnung mit der externen Einrichtung zur Kraftbeaufschlagung des Blutspeichers,
- Figur 2: eine Detailansicht der Einrichtung zur Kraftaufschlagung des Blutspeichers, und
- Figur 3: eine Ausführungsform des Blutspeichers, der die Funktion eines Luftabscheiders wahrnimmt.

Nachfolgend sind gleiche Teile mit denselben Bezugszeichen bezeichnet.

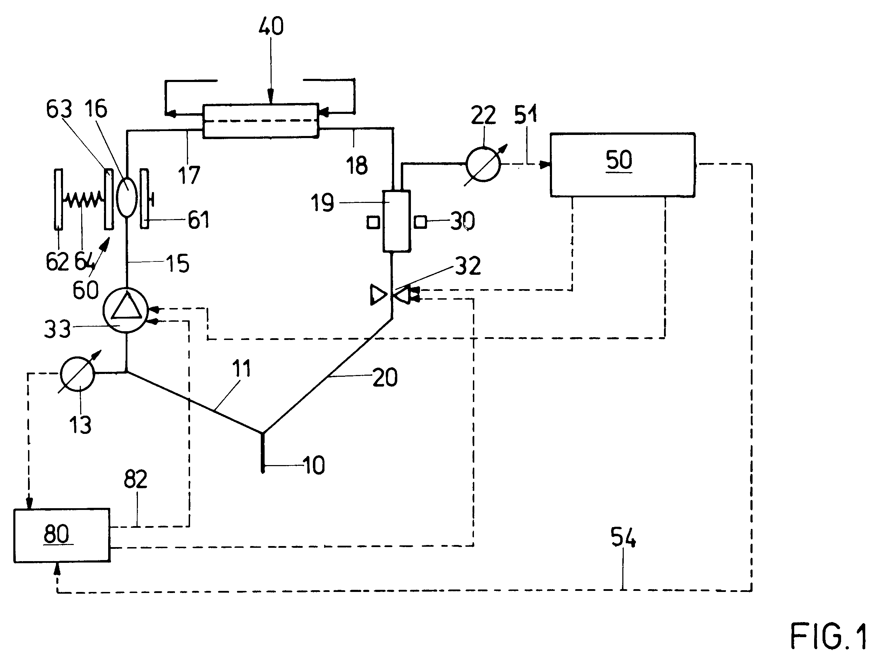

Gemäß der Anordnung nach Figur 1 wird einem Patienten über eine Hohlkanüle 10, die in ein Blutgefäß eingeschoben wird, Blut entnommen. An ihrem Ende verzweigt sich die Kanüle 10 Y-förmig. Ein Schenkel ist mit einer arteriellen Leitung 11, der andere Schenkel mit einer venösen Leitung 20 verbunden.

Die Leitung 11 führt zu der arteriellen Blutpumpe 33, an die sich eine Leitung 15 zum Blutspeicher 16 anschließt. Der Blutspeicher ist von Platten 61, 63 der externen Einrichtung 60 eingefaßt, welche den Blutspeicher 16 mit einer von seinem Füllvolumen unabhängigen Kraft beaufschlagt. Die Einrichtung 60 wird weiter unten im Einzelnen erläutert.

Vom Blutspeicher 16 führt eine Leitung 17 zu einem nur schematisch angedeuteten Dialysator 40.

Blut fließt dann in der Leitung 18 zu einer venösen Tropfkammer 19, an der ein Ultraschall-Luftdetektor 30 angeordnet ist, um ggf. im Blut befindliche Luftbläschen zu detektieren.

Stromabwärts ist eine ansteuerbare Klemme 32 vorgesehen, welche die venöse Leitung 20 abklemmen kann.

Ein arterieller Druckmonitor 13 überwacht den Druck in der Leitung 11 und leitet entsprechende Signale an die Drucküberwachungseinrichtung 80 weiter.

Eine Steuereinheit 50 übernimmt die Steuerung des Single-Needle-Verfahrens. Hierzu werden ihr über eine Leitung 54 die Signale von der Drucküberwachungseinheit 80 und über eine Leitung 51 von einem venösen Druckmonitor 22 zugeführt. Die Steuereinheit 50 steuert in Abhängigkeit von den überwachten Drücken abwechselnd die Blutpumpe 33 und die Klemme 32 an, sodaß in bekannter Weise ein intermittierender Blutfluß in der Kanüle 10 erzeugt wird.

In einer Abwandlung dieser Ausführung kann der erfindungsgemäße Blutspeicher auch stromab des Dialysators eingesetzt werden. In diesem Fall kann die venöse Tropfkammer entfallen, falls die üblicherweise an der Tropfkammer vorgesehenen Anschlüsse für die Zufuhr von Infusionen anderweitig vorgesehen werden.

Auch eine Anordnung sowohl stromauf als auch stromab des Dialysators ist möglich.

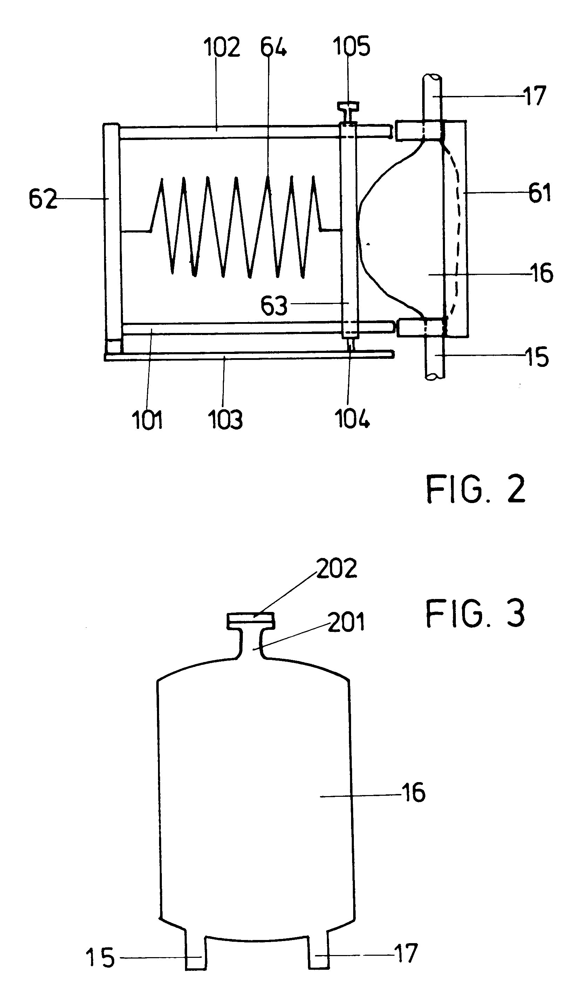

Eine Ausführungsform der Einrichtung 60 ist in Figur 2 dargestellt. Sie besteht aus einer feststehenden Platte 61, an der der Blutspeicher 16 zur Anlage kommt. Sie ist mittels Führungsstangen 101, 102 mit einer ebenfalls feststehenden Grundplatte 62 verbunden. Zwischen der Platte 61 und der Grundplatte 62 ist eine Platte 63 an den sie durchgreifenden Führungsstangen 101, 102 verschieblich gelagert. Zwischen der Grundplatte 62 und der beweglichen Platte 63 ist eine Schraubenfeder 64 angeordnet, welche die Platte 63 stets unter eine in Richtung der Platte 61 gerichteten Kraft hält.

Ein Linearpotentiometer 103 ist fest an der Grundplatte 62 montiert. Ein an der beweglichen Platte 63 angebrachter Reiter 104 wirkt mit dem Linearpotentiometer 103 als Absolutweggeber, mit dem die Position der beweglichen Platte 63 gegenüber der Grundplatte 62 bzw. der feststehenden Platte 61 erfaßt werden kann.

In der arteriellen Blutflußphase, also wenn die Klemme 32 die Leitung 20 absperrt und die Blutpumpe 33 Blut fördert, wird sich der elastische Blutspeicher 16 nach Art eines Ballons ausdehnen mit der Wirkung, daß die bewegliche Platte 63 entgegen der Kraft der Feder 64 auf die Grundplatte 62 zu verschoben wird.

Beim Umschalten von der arteriellen zu der venösen Blutflußphase wird die Blutpumpe 33 gestoppt und die Klemme 32 geöffnet. Die Blutpumpe 33, die eine übliche Rollenpumpe sein kann, klemmt dabei die Leitung 15 ab.

Aufgrund der Vorspannung der Feder 64 wird nun die bewegliche Platte 63 in Richtung der feststehenden Platte 61 gedrückt, wodurch der Blutspeicher mit einer konstanten Kraft (die insbesondere unabhängig von seinem Füllvolumen ist), beaufschlagt und entleert wird.

In der venösen Blutflußphase wird aufgrund der externen Einrichtung 60 ein im wesentlichen konstanter Blutfluß in den venösen Leitungen 18, 20 erzielt.

Die Einrichtung 60 weist im dargestellten Ausführungsbeispiel eine Arretierungsvorrichtung 105 auf, durch welche die Platte 63 in einer vorbestimmbaren Position zwischen der Grundplatte 62 und der feststehenden Platte 61 festlegbar ist. Dies ist dann von Interesse, wenn die Einrichtung 60 bei einem Betrieb der Anordnung in einem Zwei-Nadel-Verfahren nicht entfernt werden soll, um das Füllvolumen des Kreislaufs nicht zu vergrößern.

Der Blutspeicher 16 kann als einfacher Beutel aus elastischem Material ausgebildet sein. Vorteilhaft ist die Ausbildung, die in Figur 3 angedeutet ist. Hier fungiert der Beutel nicht nur als Blutspeicher, sondern auch als Luftabscheider. Er verfügt über zwei Blutanschlüsse 15, 17. Desweiteren weist er eine Öffnung oder Anschluß 201 auf, der mit einer Hydrophobmembran 202 verschlossen ist, die aber ein Entweichen von Luft aus dem Beutel zuläßt.

## Patentansprüche

1. Vorrichtung zum Reinigen des Blutes von Stoffwechselprodukten mit Hilfe eines Dialysators in einem extrakorporalen Kreislauf in einer Single-Needle-Anordnung mit einer von der Kanüle wegführenden arteriellen Leitung und einer zu dieser Kanüle hinführenden Venenleitung, mit wenigstens einer Blutpumpe zumindest in der arteriellen Leitung, mit einem Dialysator zwischen der arteriellen und der venösen Leitung, mit wenigstens einem steuerbaren Absperrorgan in einer der Leitungen stromab der Blutpumpe, mit einer Steuereinrichtung, welche die Pumpe und das Absperrorgan derart ansteuert, daß der venöse und der arterielle Blutfluß intermittieren, und mit wenigstens einem Blutspeicher in einer der beiden Leitungen, der während der arteriellen Blutflußphase mit Blut gefüllt wird,
gekennzeichnet durch,
eine, auf den Blutspeicher (16) eine im wesentlichen konstante Kraft ausübende Einrichtung (60), die bewirkt, daß sich im Inneren des Blutspeichers ein im wesentlichen von seinem Füllvolumen unabhängiger Druck einstellt.

2. Vorrichtung nach Anspruch 1, dadurch gekennzeichnet, daß die Einrichtung (60) mindestens aus einer feststehenden Platte (61) und aus einer dieser gegenüber beweglichen Platte (63) besteht, wobei die bewegliche Platte (63) mittels einer auf sie einwirkenden Feder (64) mit einer einstellbaren Kraft gegen die feststehende Platte (61) gedruckt wird und der Blutspeicher (16) zwischen beiden Platten (61, 63) angeordnet ist.

3. Vorrichtung nach Anspruch 1 oder 2, dadurch gekennzeichnet, daß der Blutspeicher (16) in Form einer elastischen Kammer ausgebildet ist.

4. Vorrichtung nach Anspruch 3, bei der die Platte (63) eine wirksame Fläche A und der Blutspeicher (16) eine im wesentlichen plane, maximale Auflagefläche B bei minimalem Füllvolumen aufweist, dadurch gekennzeichnet, daß für das Verhältnis A:B die folgende Beziehung gilt.
0,7 < A/B < 0,9.

5. Vorrichtung nach Anspruch 4, bei der die maximale Dicke des Blutspeichers bei maximalem Füllvolumen a beträgt und der Blutspeicher eine maximale Dimension b aufweist, dadurch gekennzeichnet, daß für das Verhältnis a:b die folgende Beziehung gilt:
0,08 < a/b < 0,12.

6. Vorrichtung nach einem der Ansprüche 1 bis 5, dadurch gekennzeichnet, daß die Einrichtung (60) mit einem Absolutweggeber (103, 104) versehen ist, welcher die Position der beweglichen Platte (63) gegenüber der feststehenden Platte (61) erfaßt.

7. Vorrichtung nach Anspruch 6, dadurch gekennzeichnet, daß die Einrichtung (60) aus einer feststehenden Platte (61), an welcher der Blutspeicher (16) zur Anlage kommt, aus einer feststehenden Grundplatte (62), aus mindestens einer Führungsstange (101, 102) zwischen diesen beiden Platten, aus einer durch die Führungsstange (101, 102) geführten beweglichen Platte (63), und aus einer zwischen der Grundplatte (62) und der beweglichen Platte (63) angeordneten Schraubenfeder (64) besteht, und daß der Absolutweggeber gebildet ist aus einem ortsfesten Linearpotentiometer (103) und aus einem an der beweglichen Platte (63) befestigten Reiter (104).

8. Vorrichtung nach Anspruch 1, dadurch gekennzeichnet, daß die Einrichtung (60) aus zwei in einem Rahmen schwimmend gelagerten Platten besteht, von denen jede mittels jeweils einer auf sie einwirkenden Feder mit einer einstellbaren Kraft gegen die jeweils andere Platte gedrückt wird, wobei der Blutspeicher zwischen beiden Platten angeordnet ist.

9. Vorrichtung nach Anspruch 8, dadurch gekennzeichnet, daß der Blutspeicher (16) in Form einer elastischen Kammer ausgebildet ist.

10. Vorrichtung nach Anspruch 9, bei der die Platten eine wirksame Fläche C und der Blutspeicher im wesentlichen plane, maximale Auflageflächen D bei minimalen Füllvolumen aufweist, dadurch gekennzeichnet, daß für das Verhältnis C:D die folgende Beziehung gilt:
0,7 < C/D <0,9.

11. Vorrichtung nach Anspruch 10, bei der die maximale Dicke des Blutspeichers bei maximalem Füllvolumen c beträgt und der Blutspeicher eine minimale Dimension d aufweist, dadurch gekennzeichnet, daß für das Verhältnis c:d die folgende Beziehung gilt:
0,16 < c/d < 0,24.

12. Vorrichtung nach einem der Ansprüche 1 bis 11, dadurch gekennzeichnet, daß das maximale Füllvolumen des Blutspeichers (16) durch einen einstellbaren Anschlag für die bewegliche Platte (63) bzw. für die beiden schwimmenden Platten festlegbar ist.

13. Vorrichtung nach einem der Ansprüche 1 bis 12, dadurch gekennzeichnet, daß der Blutspeicher (16) als Luftabscheider asugebildet ist und hierzu mit einer durch eine hydrophobe, luftdurchlässige Membran (202) verschlossenen Öffnung (201) versehen ist.

14. Verfahren zum Betreiben der Vorrichtung nach den Ansprüchen 1 bis 13, bei dem der Blutfluß im arteriellen und venösen Schenkel intermittierend gesteuert wird, und bei dem während der arteriellen Blutflußphase Blut in den mindestens einen Blutspeicher gepumpt wird, dadurch gekennzeichnet, daß zumindest während der venösen Blutflußphase der Blutspeicher (16) von einer externen Einrichtung (60) so mit Kraft beaufschlagt wird, daß sich im Inneren des Blutspeichers (16) ein im wesentlichen von seinem Füllvolumen unabhängiger Druck einstellt.

15. Verfahren nach Anspruch 14, dadurch gekennzeichnet, daß der Blutspeicher (16) während der arteriellen Blutflußphase entgegen der von der Einrichtung (60) erzeugten Kraft mit Blut gefüllt wird, wodurch die Einrichtung vorgespannt wird, und daß der Blutspeicher (16) während der venösen Blutflußphase unter Abbau der Vorspannung entleert wird.

## Claims

1. A device for the purification of blood from metabolic products by means of a dialyzer positioned in an extracorporeal circulation in a single needle system, said device comprising
an arterial conduction running from the canule and a venous conduction running to the said canule,
at least one blood pump positioned in the arterial conduction at the least, a dialyzer between the arterial and the venous conduction,
at least one controllable shut-off device in one of the conductions downstream the blood pump,
a control device driving the pump and the shut-off device so as to cause the venous and arterial blood flow to intermit, and
at least one blood reservoir positioned in one of the two conductions and being filled with blood in the course of the arterial phase of blood flow,
characterized by
an installation (60) exercising an essentially constant force on the blood reservoir (16) so as to generate a pressure inside said blood reservoir which is essentially independent of the actual fill contents thereof.

2. A device of claim 1, characterized in that the installation (60) comprises at least a stationary plate (61) and a plate (63) in movable relationship therewith, said movable plate (63) pressing with an adjustable force against said stationary plate (61) by means of a spring (64) engaging with said movable plate, and the blood reservoir (16) being positioned between said two plates (61,63).

3. A device of claims 1 or 2, characterized in that the blood reservoir (16) is developed in form of an elastic chamber.

4. A device of claim 3, wherein the plate (63) has an effective area (A) and, when having minimum fill contents, the blood reservoir (16) has an essentially plane maximum engaging area (B), characterized in that the ratio A:B shows the following relationship:
0.7 < A/B < 0.9.

5. A device of claim 4, wherein the maximum depth of the blood reservoir is a when having maximum fill contents a, and the blood reservoir has a maximum dimension b, characterized in that the ratio a:b shows the following relationship:
0.08 < a/b < 0.12.

6. A device of one of claims 1 through 5, characterized in that the installation (60) comprises a true distance meter (103,104) indicating the position of the moveable plate (63) as against stationary plate (61).

7. A device of claim 6, characterized in that the installation (60) comprises a stationary plate (61) against which nestles the blood reservoir (16), a stationary base plate (62), at least one motion rod (101,102) between the said two plates, a moveable plate (63) guided by the said motion rod (101,102), and a helical spring (64) disposed between the said base plate (62) and the said moveable plate (63) and in that the true distance meter is built up of a stationary linear potentiometer (103) and of a rider (104) mounted on the said moveable plate (63).

8. A device of claim 1, characterized in that the installation (60) comprises two plates floatingly positioned in a frame, each of which being pressed against the right opposite plate with adjustable force by means of a spring acting on said plate, the blood reservoir being disposed between the two plates.

9. A device of claim 8, characterized in that the blood reservoir (16) is developed in form of an elastic chamber.

10. A device of claim 9, wherein the plates have an effective area C and the blood reservoir has an essentially plane, maximum engaging area D while having minimum fill contents, characterized in that the ratio C:D has the following relationship:
0.7 < C/D < 0.9.

11. A device of claim 10 wherein, while having maximum fill contents, the maximum thickness of the blood reservoir is c and the blood reservoir has a minimum dimension d, characterized in that the ratio c:d has the following relationship:
0.16 < c/d < 0.24.

12. A device of one of claims 1 to 11, characterized in that the maximum fill contents of the blood reservoir (16) can be defined by an adjustable stopper for the moveable plate (63) or for the two floating plates, respectively.

13. A device of one of claims 1 to 12, characterized in that the blood reservoir (16) is developed as an air separator and has for this purpose a port (201) being closed by a hydrophobic air permeable membrane (202).

14. A method for operating the device of claims 1 through 13, wherein the blood flow in the arterial and the venous leg is controlled intermittently and wherein, during the arterial blood phase, the blood is pumped into the at least one blood reservoir, characterized in that, at least in the course of the venous blood phase, a force from an external installation (60) is exerted onto the blood reservoir (16) so as to generate a pressure in the interior of the blood reservoir (16) that is essentially independent from its fill contents.

15. A method of claim 14, characterized in that, in the course of arterial blood phase, the blood reservoir (16) is filled with blood against the force generated by the said installation (60) thereby prestressing the said installation and in that, in the course of the venous blood phase, the blood reservoir (16) empties in declining the prestress.

## Revendications

1. Dispositif d'épuration du sang de produits du métabolisme à l'aide d'un dialyseur dans une circulation extracorporelle par un dispositif à une aiguille avec une conduite artérielle partant de la canule et une conduite veineuse amenant vers cette canule, avec au moins un pompe à hémodialyse, du moins dans la conduite artérielle, avec un dialyseur entre les conduites artérielle et veineuse, avec au moins un organe de retenue commandable dans une des conduites en aval de la pompe à hémodialyse, avec un organe de commande qui excite la pompe et l'organe de retenue de sorte que les flux sanguins artériel et veineux alternent, et avec au moins un accumulateur de sang dans une des deux conduites, qui est rempli de sang lors de la phase artérielle du flux sanguin, caractérisé par un appareillage (60) exerçant une force essentiellement constante sur l'accumulateur de sang (16) qui donne lieu à l'intérieur de l'accumulateur de sang à la formation d'une pression essentiellement indépendante de son volume de remplissage.

2. Dispositif selon la revendication 1, caractérisé en ce que l'appareillage (60) se compose au moins d'une plaque fixe (61) et d'une plaque mobile (63) par rapport à celle-ci, la plaque mobile (63) étant pressée contre la plaque fixe (61), avec une force réglable, au moyen d'un ressort agissant sur elle (64) et l'accumulateur de sang (16) étant disposé entre ces deux plaques (61, 63).

3. Dispositif selon la revendication 1 ou 2, caractérisé en ce que l'accumulateur de sang (16) est conçu sous la forme d'une chambre élastique.

4. Dispositif selon la revendication 3, dans lequel la plaque (63) présente une surface active A et l'accumulateur de sang (16) présente une surface d'appui B maximale essentiellement plane en cas de volume de remplissage minimum, caractérisé en ce que le rapport A:B répond à l'équation suivante:
0,7 < A/B < 0,9.

5. Dispositif selon la revendication 4, dans lequel l'épaisseur maximale de l'accumulateur de sang en cas de volume de remplissage maximal s'élève à a et l'accumulateur de sang présente une dimension maximale b, caractérisé en ce que le rapport a:b répond à l'équation suivante:
0,08 < a/b < 0,12.

6. Dispositif selon l'une quelconque des revendications 1 à 5, caractérisé en ce que l'appareillage (60) est pourvu d'un capteur de course absolue (103, 104), qui saisit la position de la plaque mobile (63) par rapport à la plaque fixe (61).

7. Dispositif selon la revendication 6, caractérisé en ce que l'appareillage (60) se compose d'une plaque fixe (61) contre laquelle l'accumulateur (16) de sang est appliqué, d'un support fixe (62), d'au moins une tige conductrice (101, 102) entre ces deux plaques, d'une plaque mobile (63) guidée par la tige conductrice (101, 102) et d'un ressort à boudin (64) disposé entre le support (62) et la plaque mobile (63), et en ce que le capteur de course absolue est formé d'un potentiomètre linéaire fixe (103) et d'un cavalier fixé (104) à la plaque mobile (63).

8. Dispositif selon la revendication 1, caractérisé en ce que l'appareillage (60) se compose de deux plaques flottantes dans un cadre, dont chacune est pressée contre respectivement l'autre plaque avec une force réglable au moyen d'un ressort agissant respectivement sur chacune d'elles, l'accumulateur de sang étant disposé entre les deux plaques.

9. Dispositif selon la revendication 8, caractérisé en ce que l'accumulateur de sang (16) est conçu sous la forme d'une chambre élastique.

10. Dispositif selon la revendication 9, dans lequel les plaques présentent une surface active C et l'accumulateur présente des surfaces d'appui D maximales essentiellement planes en cas de volumes de remplissage minimum, caractérisé en ce que le rapport C:D répond à l'équation suivante:
0,7 < C/D < 0,9.

11. Dispositif selon la revendication 10, dans lequel l'épaisseur maximale de l'accumulateur de sang s'élève à c en cas de volume de remplissage maximum et l'accumulateur de sang présente une dimension minimum d, caractérisé en ce que le rapport c:d répond à l'équation suivante:
0,16 < c/d < 0,24.

12. Dispositif selon l'une quelconque des revendications 1 à 11, caractérisé en ce que le volume de remplissage maximal de l'accumulateur de sang (16) peut être fixé par une butée réglable pour la plaque mobile (63) ou pour les deux plaques flottantes.

13. Dispositif selon l'une quelconque des revendications 1 à 12, caractérisé en ce que l'accumulateur de sang (16) est conçu comme un séparateur d'air et est pourvu à cet effet d'une ouverture (201) fermée par une membrane (202) hydrophobe perméable à l'air.

14. Procédé mettant en oeuvre le dispositif selon les revendications 1 à 13, dans lequel le flux sanguin est commandé de manière intermittente dans les branches artérielle et veineuse et dans lequel est pompé du sang dans au moins un accumulateur de sang pendant la phase artérielle du flux sanguin, caractérisé en ce qu'au moins pendant la phase veineuse du flux sanguin, l'accumulateur de sang (16) est alimenté par une force d'un appareillage externe (60) et en ce qu'à l'intérieur de l'accumulateur de sang (16) intervient une pression essentiellement indépendante de son volume de remplissage.

15. Procédé selon la revendication 14, caractérisé en ce que l'accumulateur de sang (16) est rempli de sang pendant la phase artérielle de flux de sang contre la force produite par l'appareillage (60), ce qui place l'appareillage sous précontrainte et en ce que l'accumulateur de sang (16) est vidé pendant la phase veineuse du flux sanguin par détente de la précontrainte.
